# EUROPÄISCHE PATENTSCHRIFT

(11) **EP 4 590 215 B1**
(45) Veröffentlichungstag und Bekanntmachung des Hinweises auf die Patenterteilung: **11.03.2026**
(21) Anmeldenummer: 24820368.9
(22) Anmeldetag: 04.12.2024
(51) Int. Cl.: A61B 17/29

(54) **CHIRURGISCHES MINIMALINVASIVE INSTRUMENT**
MINIMALLY INVASIVE SURGICAL INSTRUMENT
INSTRUMENT CHIRURGICAL MINIMALEMENT INVASIF

(30) Priorität: 08.12.2023 DE 102023134397
(43) Veröffentlichungstag der Anmeldung: 30.07.2025
(73) Patentinhaber: Aesculap AG, 78532 Tuttlingen (DE)
(72) Erfinder: MELCER, Marco, 72469 Meßstetten (DE); REUTER, Michael, 88637 Leibertingen-Thalheim (DE)
(74) Vertreter: Winter, Brandl - Partnerschaft mbB
(86) Internationale Anmeldenummer: PCT/EP2024/084642
(87) Internationale Veröffentlichungsnummer: WO 2025/119962

(56) Entgegenhaltungen:
- US-A- 5 330 502
- US-A- 5 626 587
- US-A1- 2016 361 107
- US-A1- 2017 071 618
- US-B2- 11 510 669

## Beschreibung

### Technisches Gebiet

Die vorliegende Offenbarung betrifft ein medizinisches Instrument für minimalinvasive Chirurgie, insbesondere ein laparoskopisches Instrument, mit verbessertem Aufbau, insbesondere mit verbesserter Artikulationsmechanik.

### Hintergrund der Offenbarung

Aus dem Stand der Technik sind medizinische Instrumente für minimalinvasive Chirurgie bekannt. Solche Instrumente haben für gewöhnlich einen länglichen (schaftartigen) Aufbau, mit einem proximalen Ende/Endabschnitt, an welchem ein Benutzer, meist ein Chirurg, das Instrument handhabt, und mit einem distalen Ende/Endabschnitt (auch "Anwendungsende" genannt), an dem eine Funktionseinheit (Effektor) angebracht ist, beispielsweise ein Schneid- und/oder Versiegelungswerkzeug (engl. "seal and cut instrument"). Zwischen dem proximalen Ende und dem distalen Ende verläuft typischerweise ein Schaftrohr mit geringem Durchmesser, welches beispielsweise durch eine Trokarhülse (oder Arbeitskanal eines Endoskops) in den Körper eines Patienten eingeführt werden kann.

Auf diese Weise kann die Funktionseinheit im Inneren eines Patienten zum Einsatz gebracht werden, während das distale Ende des Instruments zur Handhabung durch den Benutzer außen verbleibt. Diese Art der Chirurgie ist in dem Sinne minimalinvasiv, dass nur die minimal mögliche Öffnung des Körpers des Patienten bereitgestellt werden muss.

Aufgrund des länglichen Aufbaus des Instruments besteht eine Herausforderung darin, die sehr akkuraten Einstellungen und Betätigungen des Benutzers an der proximalen Seite zuverlässig und präzise in Aktionen der Funktionseinheit umzusetzen. Während einerseits eine robuste Ausführung des Instruments dafür wichtig ist, werden andererseits eine kompakte Bauform sowie ein geringes Gewicht und eine leichte Handhabung des Instruments geschätzt.

Darüber hinaus wird häufig eine Artikulierbarkeit eines distalen Teils des Instruments verlangt, welches beispielsweise als Gelenkteil bezeichnet werden kann. Diese Artikulierbarkeit des Gelenkteils kann der Ausrichtung einer Funktionseinheit des Instruments während eines chirurgischen Eingriffs dienen.

### Stand der Technik

Aus dem Stand der Technik sind derartige, wie vorstehend erläuterte chirurgische Instrumente der minimalinvasiven Bauart bekannt, mit einer proximalen Handhabe, an die sich ein Instrumentenschaft (starr oder flexibel) anschließt. Häufig sind der Instrumentenschaft und die Handhabe über eine Drehkopplung miteinander verbunden oder verbindbar, um den Schaft relativ zur Handhabe um dessen Längsachse zu drehen. Am distalen Ende des Schafts ist ein Instrumentenkopf drehbar und oder abkippbar gelagert, an dem wiederum ein Effektor montiert, insbesondere gelagert ist.

Zur Betätigung des Instrumentenkopfs sind an der vorzugsweise als Handgriff ausgebildeten Handhabe Aktuatoren (beispielsweise Knöpfe/Tasten, Hebel, Handräder etc.) vorgesehen, die über einen Getriebezug mit dem Instrumentenkopf wirkverbunden sind. Ferner sind Aktuatoren zur Betätigung/zum Betreiben des Effektors an der Handhabe angeordnet.

Der Getriebezug und die Verbindungsleitungen zum Effektor sind in der Regel innerhalb des Instrumentenschafts verlegt, der hierfür rohrförmig ausgestaltet ist. Grundsätzlich kann der Getriebezug eine Rotations- oder eine Schub-/Zugstange sein, deren jeweilige Bewegung in eine Schwenk- und/oder Drehbewegung des Instrumentenkopfs transformiert wird. Hierfür ist ein Transformationsmechanismus beispielsweise in Form von Kraftumlenkelementen oder dergleichen im Bereich des distalen Instrumentenkopfs erforderlich, der in der Regel mechanisch kompliziert und aufwändig ist. Da bei einem solchen Bewegungs-Transformationsmechanismus immer mehrere Teile (seriell, d.h. in Richtung des Getriebezugs) zusammenwirken müssen, ergibt sich daraus zwangsläufig eine Toleranzkette, die im Endergebnis dazu führt, dass die Handhabung des Instruments für den Bediener schwammig und ungenau wird. Auch ist mit einer höheren Störanfälligkeit zu rechnen.

US 11 510 669 B2 offenbart ein handgehaltenes chirurgisches Instrument mit einem Griffgehäuse, einem länglichen Schaftabschnitt, der so eingerichtet ist, dass er sich distal relativ zum Griffgehäuse erstreckt, und einem chirurgischen Endeffektor, der so eingerichtet ist, dass er mit einem distalen Endabschnitt des Schaftabschnitts gekoppelt werden kann. Eine Gelenkschraube ist mit dem chirurgischen Endeffektor funktionsfähig verbunden, und eine Gelenkmutter ist um die Gelenkschraube herum angeordnet und mit dieser funktionsfähig verbunden. Die Gelenkschraube ist so eingerichtet, dass sie sich als Reaktion auf eine Drehung der Gelenkmutter verschiebt, wodurch die Gelenkschraube den chirurgischen Endeffektor zwischen einer parallelen Ausrichtung relativ zum Schaftabschnitt und einer nicht parallelen Ausrichtung relativ zum Schaftabschnitt bewegt.

US 5 330 502 A offenbart ein endoskopisches Instrument wie einen Dissektor, eine Schere oder einen Greifer, bei dem ein Schaft vorgesehen ist, der eine Längsachse des Instruments definiert. Der Schaft kann sich um den Griffteil des Instruments drehen. Dieser Mechanismus ermöglicht auch die Artikulation der Endeffektoren in Bezug auf die Längsachse des Schafts. Diese Artikulation wird durch eine Winkelung des Endeffektors in Bezug auf den Schaft erreicht. Auch wird ein Verriegelungsmechanismus offenbart, der es ermöglicht, dass der Rotationsaspekt der Vorrichtung den gesamten Gelenkmechanismus dreht.

US 2017 0 071 618 A1 offenbart ein steuerbares laparoskopisches Instrument, mit einem Übertragungsteil, einer ersten Scheibe, einer zweiten Scheibe, einem Biegeteil, einer dritten Scheibe, einem Arbeitsteil, einem Griffteil und einem Gehäuse. Die erste Scheibe ist mit einem Außenrohr so verbunden, dass die Drehung der ersten Scheibe auf einer ersten Achse, entlang derer sich das Außenrohr erstreckt, das Außenrohr entsprechend auf der ersten Achse in Drehung versetzt. Die zweite Scheibe ist mit einem Innenrohr so gekoppelt, dass die Drehung der zweiten Scheibe auf der ersten Achse das Innenrohr entsprechend entlang der ersten Achse bewegt. Die dritte Scheibe ist an einem ersten Ende des Übertragungsteils angeordnet. Eine zentrale Übertragungsstange, die sich entlang der ersten Achse erstreckt, ist mit der dritten Scheibe gekoppelt. Die zentrale Übertragungsstange ist mit der dritten Scheibe so gekoppelt, dass die Drehung der dritten Scheibe auf der ersten Achse die zentrale Übertragungsstange antreibt, um sich entsprechend auf der ersten Achse zu drehen.

US 2016 0 361 107 A1 offenbart ein chirurgisches Instrument mit einem länglich geformten äußeren Instrumentenschaft. Ein proximaler Endabschnitt des äußeren Schafts ist operativ mit einem Schaftdrehknopf verbunden, der im Laufabschnitt einer proximalen Griffanordnung gelagert ist. Der Knopf ist so angebracht, dass er eine axiale Drehung des äußeren Instrumentenschafts zusammen mit einem Betätigungsschaft um dessen Längsachse bewirkt.

US 5 626 587 A offenbart einen endoskopischen chirurgischen Klammernahtgerät mit einem röhrenförmigen Schaft, der relativ zu einem Griff um eine Längsachse gedreht werden kann. Die Schaftdrehung wird am Griff durch Drehen eines Knopfes bewirkt, der am Griff angebracht ist und mit dem röhrenförmigen Schaft in Eingriff steht.

### Zusammenfassung der Offenbarung

Es sind daher die Aufgaben und Ziele der vorliegenden Offenbarung, die oben genannten Herausforderungen aus dem Stand der Technik zu bewältigen und die genannten bestehenden Bedürfnisse zu befriedigen, und insbesondere ein verbessertes artikulierbares Instrument für die minimalinvasive Chirurgie, bevorzugt ein laparoskopisches Instrument, bereitzustellen, welches eine geringe Bauform und gute Handhabbarkeit mit einer präzisen Bedienbarkeit verbindet.

Die Aufgaben der vorliegenden Offenbarung werden durch die Merkmale des Anspruchs 1 gelöst.

Demnach stellt die Erfindung ein Instrument für minimalinvasive Chirurgie, vorzugsweise für die Laparoskopie, bereit, aufweisend:
ein Schaftrohr;
ein Gelenkteil, welches an einem distalen Ende/Endabschnitt des Schaftrohrs derart gelenkig mit dem Schaftrohr verbunden ist, dass das Gelenkteil bezüglich des Schaftrohrs um eine (rechtwinklig zur Schaftrohrachse sich ersteckende) Drehachse schwenkbar (oder: artikulierbar) ist;
einen proximalen Aktuatormechanismus (15, 16); und
ein Innenteil, welches teilweise in das Schaftrohr (und optional teilweise in das Gelenkteil) eingesetzt ist und, über den proximalen Aktuatormechanismus, innerhalb des Schaftrohrs axial bewegbar ist;
wobei das Innenteil an seinem distalen Ende derart mit dem Gelenkteil gekoppelt ist, dass eine axiale Bewegung des Innenteils eine Schwenkung (oder: Artikulation) des Gelenkteils um die Drehachse bewirkt.

Ein Grundgedanke der vorliegenden Offenbarung ist darin zu sehen, dass durch eine geschickte Ausführung eines Innenteils (oder "Inlays") sowie einen distalen Artikulationsmechanismus als auch einen proximalen Aktuatormechanismus, welche mit dem Innenteil zusammenwirken, sowohl mechanische Betätigungen des Instruments ermöglicht als auch strukturelle Stabilität bereitgestellt werden. Weiterhin werden sowohl eine Bauteilreduktion als auch eine Vereinfachung der Mechanik realisiert.

Die Begriffe "proximal" und "distal" beziehen sich hierin stets auf die Sichtweise des Benutzers des Instruments, welcher das Instrument an seiner proximalen Seite handhabt, damit eine Funktionseinheit an dessen distalem Ende mit dem Patienten interagieren kann.

Vorliegend wird unter einer "axialen" Bewegung eine Bewegung entlang einer Längsachse verstanden, insbesondere eine Bewegung des länglichen Innenteils innerhalb und bezüglich des länglichen Schaftrohrs. Die axiale Richtung kann auch als "longitudinale Richtung" bezeichnet werden. Eine "transversale" Richtung ist senkrecht zu der Längsachse angeordnet.

Vorteilhafte Ausführungsformen sind in den Unteransprüchen beansprucht und werden insbesondere nachfolgend erläutert.

Das Schaftrohr kann vorzugsweise im Bereich eines proximalen Endes ein Außengewinde aufweisen (das Gewinde geht bevorzugt nicht bis ganz zum proximalen Schaftende, ist aber möglichst nah an dem proximalen Schaftende vorgesehen), in welches ein Innengewinde eines Aktuatorelements eingreift. Das Aktuatorelement kann mit einem Mitnehmer des Instruments derart gekoppelt sein, dass sich bei einer Rotationsbewegung des Aktuatorelements um das Schaftrohr (oder: um das Außengewinde des Schaftrohrs) der Mitnehmer axial bewegt, insbesondere ausschließlich axial. Eine (nach innen gerichtete) Nase des Mitnehmers kann dazu eingerichtet sein, durch eine Öffnung in dem Schaftrohr in eine Tasche (oder: Ausnehmung) in dem Innenteil einzugreifen und bei einer axialen Bewegung des Mitnehmers eine axiale Bewegung des Innenteils zu bewirken. Auf diese Weise kann an dem proximalen Ende des Instruments eine platzsparende Rotationsbewegung in eine gewünschte axiale Bewegung des Innenteils umgesetzt werden. Diese axiale Bewegung wiederum wird erfindungsgemäß in eine Schwenkung (oder: Artikulation) des Gelenkteils umgesetzt.

Die Kopplung von Schaftrohr, Aktuatorelement, Mitnehmer und Innenteil kann besonders bevorzugt selbstsperrend ausgeführt sein. Somit wird die geschwenkte Stellung des Gelenkteils vorteilhaft beibehalten, bis sie - durch erneute rotatorische Bewegung des Aktuatorelements - wieder verändert wird. Dies ermöglicht eine hohe Präzision in der Führung einer Funktionseinheit am distalen Ende des Instruments, insbesondere am distalen Ende des Gelenkteils.

Das Aktuatorelement kann vorteilhaft an seiner Außenseite mit einer Zahnradstruktur (oder: als Zahnrad) ausgebildet sein. Auf diese Weise kann eine Benutzereingabe rotatorisch erfasst oder umgesetzt werden und in einer Weise auf das Aktuatorelement übertragen werden.

Das (bevorzugt mit der Zahnradstruktur ausgebildete) Aktuatorelement kann mehrstückig ausgebildet und zusammengesetzt um das Schaftrohr angeordnet herum sein. Die mehrstückige Ausbildung hat Vorteile bei der Herstellung, z.B. im Spritzgussverfahren, aber auch bei der Montage um das Schaftrohr, da das Aktuatorelement somit nicht auf das Schaftrohr aufgefädelt werden muss sondern in einem beliebigen Montageschritt um das Schaftrohr gelegt werden kann. Das mehrstückig ausgebildete Aktuatorelement kann in dem Instrument beispielsweise durch einen umlaufenden Sicherungsring und/oder einen Teil des Mitnehmers, etwa einen Ringvorsprung des Mitnehmers, zusammengehalten werden.

Gemäß einer bevorzugten Ausführungsform weist das Innenteil an seinem distalen Ende mindestens ein Langloch auf, durch welches eine mit dem Gelenkteil gekoppelte Achse geführt ist, welche von der Drehachse des Gelenkteils beabstandet und zu dieser parallel angeordnet ist. Besonders bevorzugt weist das Innenteil an seinem distalen Ende zwei (oder noch mehr) zueinander parallele und mit einander fluchtende Langlöcher auf, durch welche die mit dem Gelenkteil gekoppelte Achse (die beispielsweise fix mit dem Gelenkteil verbunden sein kann) geführt ist. Auf diese Weise ist die Schwenkbewegung noch besser geführt und somit noch stabiler. Alternativ zu mehreren Langlöchern kann auch ein einzelnes Langloch in Form einer Durchgangsbohrung mit langloch-förmigem Querschnitt ausgebildet sein.

Die (beispielsweise fix mit dem Gelenkteil verbundene) Achse, welche durch das mindestens eine Langloch des Innenteils geführt ist, ist vorteilhaft durch einen transversal angeordneten zylindrischen Stift gebildet, welcher wiederum durch eine jeweilige kreisförmige Durchgangsbohrung durch einen jeweiligen Arm von zwei proximalen Armen des Gelenkteils hindurchgeführt ist.

Bevorzugt weist das Innenteil zumindest abschnittsweise mindestens eine entlang seiner Längsachse verlaufende Ausnehmung, insbesondere Längsnut auf, in welcher ein Kraftübertragungselement (besonders bevorzugt ein Zugband für eine Betätigung eines Maulteils des Instruments) und/oder eine Klinge und/oder eine elektrische Versorgungs- und/oder Signalleitung angeordnet ist. Das Innenteil kann mehrere entlang der Längsachse verlaufende Ausnehmungen, insbesondere Längsnuten, aufweisen, wobei in einer oder mehreren davon mindestens ein Zugband und/oder in einer oder mehreren anderen davon mindestens eine elektrische Versorgungs- und/oder Steuerleitung und/oder in einer oder mehreren anderen davon mindestens eine Klinge angeordnet ist. Die jeweilige Ausnehmung, insbesondere Längsnut, kann hier jeweils bevorzugt eine Führung für das darin angeordnete Element darstellen (oder: bereitstellen).

Eine elektrische Versorgungsleistung kann beispielweise dazu eingerichtet sein, ein Wechselstromsignal (HF-Signal) zum Versorgen eines wechselstrombetriebenen Versiegelungswerkzeugs zu transportieren, oder einen Gleichstrom zum Versorgen und/oder Steuern eines Elektromotors.

Unter einer Längsnut ist hier insbesondere eine längliche Ausnehmung an einer Außenseite eines länglichen Objekts zu verstehen, die sich entlang (d.h. parallel zu) der Längsachse des länglichen Objekts (z.B. des länglich ausgebildeten Innenteils) erstreckt, bevorzugt über einen Großteil der länglichen Ausdehnung des länglichen Objekts (d.h. über 50% der länglichen Ausdehnung, über 75% der länglichen Ausdehnung oder sogar mehr). Sofern nichts anderes unmittelbar ersichtlich ist, bezieht sich die Bezeichnung "Längs- in der vorliegenden Offenbarung stets auf eine Längsachse, die sich von proximalseitig bis distalseitig erstreckt.

Das Innenteil (oder: Inlay) erfüllt somit eine Doppelfunktion in dem vorliegenden Erfindungskonzept: einerseits ist es Teil eines Kraftübertragungsmechanismus', welcher eine Aktuierung des proximalen Aktuatormechanismus' auf das Gelenkteil überträgt, um dieses zu schwenken (oder: zu artikulieren). Andererseits ist es dazu ausgelegt, Komponenten oder Elemente des Instruments, die längsweise (d.h. entlang der Längsachse des Instruments) verlaufen, zu führen.

Vorteilhaft weist das Instrument außerdem an einem distalen Ende des Gelenkteils eine Funktionseinheit und/oder an dem proximalen Ende des Schaftrohrs eine Handhabungseinheit auf. Die Handhabungseinheit kann in den proximalen Aktuatormechanismus integriert sein oder umgekehrt. Bevorzugt ist die Funktionseinheit mittels der Handhabungseinheit betätigbar. Bei der Funktionseinheit kann es sich beispielsweise um ein Schneid- und/oder Versiegelungswerkzeug, um ein Maulteil und/oder dergleichen handeln.

### Figurenliste

Die vorliegende Offenbarung wird nachfolgend anhand bevorzugter Ausführungsformen unter Bezugnahme auf die begleitenden Figuren näher erläutert. Es zeigen:
Fig. 1 eine schematische dreidimensionale Ansicht eines Abschnitts eines (medizinischen) Instruments gemäß einer Ausführungsform der vorliegenden Erfindung;
Fig. 2 eine schematische dreidimensionale Ansicht eines Details des Abschnitts aus Fig. 1 aus einer anderen Richtung;
Fig. 3 eine weitere schematische dreidimensionale Ansicht eines weiteren Details des Abschnitts aus Fig. 1 aus noch einer anderen Richtung;
Fig. 4 eine schematische Seitenansicht des Abschnitts aus Fig. 1 bei teilweiser Ausblendung von einzelnen Elementen;
Fig. 5 eine schematische Querschnittsansicht durch ein Innenteil des Instruments aus Fig. 1-4;
Fig. 6 eine schematische Längsschnittansicht eines proximalen Abschnitts des Instruments aus den Fig. 1-5;
Fig. 7, 7a, 7b schematische Ansichten eines Aktuatorelements in Form einer Spindelmutter mit Außenverzahnung gemäß Fig. 6;
Fig. 8, 8a eine schematische übersichtsartige Längsschnittansicht und Seitenansicht des Instruments aus Fig. 1-7 und
Fig. 9 eine Seitenansicht des (medizinischen) Instruments

Die Figuren sind schematischer Natur und sollen nur dem Verständnis der vorliegenden Offenbarung dienen. Gleiche Elemente sind mit denselben Bezugszeichen versehen.

### Detaillierte Beschreibung bevorzugter Ausführungsformen

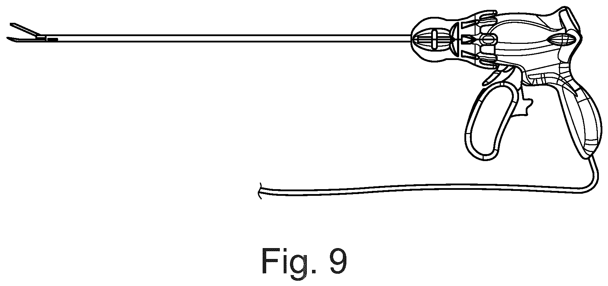

Fig. 9 zeigt den grundsätzlichen Aufbau eines chirurgischen Instruments der minimalinvasiven Bauart gemäß der Offenbarung und insbesondere ein sogenanntes laparoskopisches (elektrisches) Versiegelungs- und Schneidinstrument.

Dieses hat einen proximalen Handgriff mit einer Anzahl darin verbauter Betätigungselemente und/oder Aktuatoren (z.B. Betätigungshebel zum Öffnen und Schließen eines distalen Maulteils, Trigger zur Strombeaufschlagung des Maulteils, etc.), an dessen distalem Endabschnitt ein rohrförmiger Instrumentenschaft montiert oder montierbar ist. Der Instrumentenschaft hat einen distalen Endabschnitt, an welchem ein Instrumentenkopf abwinkelbar / schwenkbar gelagert / anscharniert ist. Der Instrumentenkopf weist einen Effektor, vorliegend in Form zweier scheren- / pinzetten- / zangenförmig bewegbarer (Gewebe-) Eingriffsbranchen (Maulteil) auf, die entsprechend am Instrumentenkopf gelagert/anscharniert sind. Beispielsweise können die Eingriffsbranchen in ihrem jeweiligen Mittenabschnitt über einen Scharnierstift miteinander gekoppelt sein, wobei der Scharnierstift ferner im Instrumentenkopf befestigt oder gelagert ist. Die distalen Abschnitte der Eingriffsbranchen (distal zum Scharnierstift) sind mit Gewebeeingriffselementen versehen oder ausgebildet beispielsweise Elektroden oder Elektrodenbereiche, um ein dazwischen eingeklemmtes Gewebe zu versiegeln (und/oder zu schneiden) und/oder eine Schneidklinge, um Gewebe zu durchtrennen. Die proximalen Abschnitte der Eingriffsbranchen (proximal zum Scharnierstift) sind mit Anlenkelementen versehen oder ausgebildet, beispielsweise Ösen, Gabel, etc., an denen jeweils ein Zug-/ oder Druckseil fixiert ist. Schließlich sind elektrische Leitungen in/an dem Instrumentenkopf verlegt, welche an den Elektroden oder mit den elektrischen Abschnitten der Branchen angeschlossen/elektrisch verbunden sind.

Sowohl das Zug-/Druckseil wie auch die elektrischen Leitungen sind innerhalb des Instrumentenschafts nach proximal zum Instrumentenhandgriff geführt und dort mit den entsprechenden Betätigungselementen / Aktuatoren für ein wahlweises Abwinkeln des Instrumentenkopfs, Öffnen und Schließen des Effektors (vorliegend Maulteils) und ein Aktuieren der Elektrode(n) verbunden/gekoppelt.

Insoweit entspricht der grundsätzliche Aufbau des chirurgischen Instruments dem eingangs beschriebenen Stand der Technik, wobei dieser Aufbau beispielsweise des Handgriffs oder des Instrumentenkopfs natürlich im Rahmen des bekannten Stands der Technik auch abgewandelt sein kann.

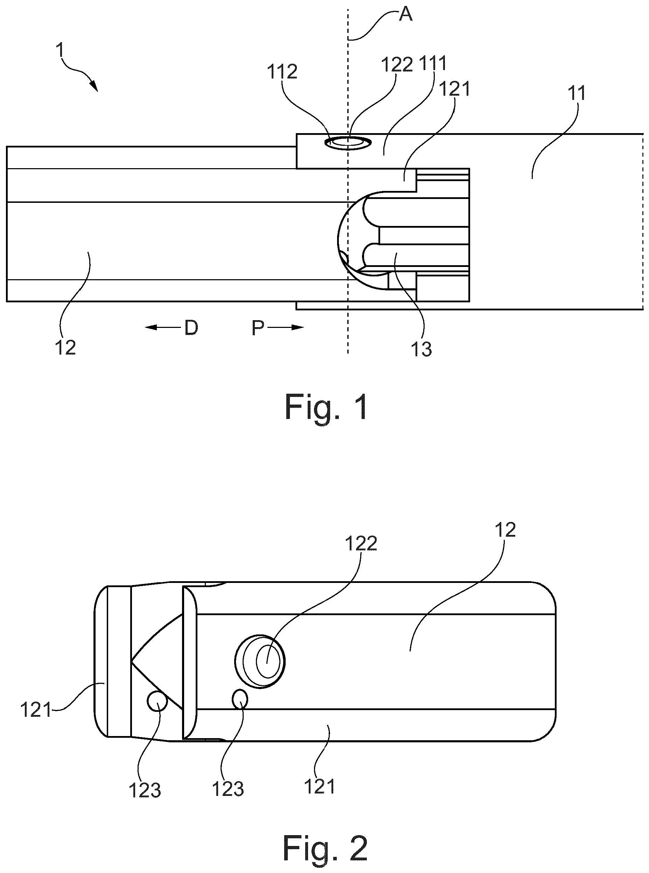

Fig. 1 zeigt eine schematische dreidimensionale Ansicht eines distalen Endabschnitts des chirurgischen/medizinischen Instruments 1 gemäß einer Ausführungsform der vorliegenden Offenbarung, in welchem ein Gelenk angeordnet ist.

Gemäß einem bevorzugten Ausführungsbeispiel der vorliegenden Offenbarung hat dieses Instrument 1 für minimalinvasive Chirurgie, vorzugsweise für die Laparoskopie, prinzipiell die folgenden Bauteile:
ein Schaftrohr 11, das an seinem proximalen Endabschnitt mit einem Griffstück gekoppelt oder koppelbar ist;
ein, einen Instrumenten-Effektor ausbildendes oder lagerndes Gelenk-/Schwenkteil 12, welches an einem distalen Endabschnitt des Schaftrohrs 11 derart gelenkig mit dem Schaftrohr 11 verbunden/gekoppelt ist, dass das Gelenk-/Schwenkteil 12 bezüglich des Schaftrohrs 11 um eine senkrecht zur Schaftrohr-Längsachse L ausgerichtete Drehachse A schwenkbar ist; und
ein inneres, stabförmiges Betätigungsteil/Innenteil 13, welches zumindest teilweise in das Schaftrohr 11 eingesetzt und darin gelagert ist, wofür die Innenwand des Schaftrohrs 11 eine Axialgleitführung für das Betätigungsteil 13 bildet oder aufweist, wobei das Betätigungsteil 13 über einen proximalen Getriebe-/Aktuatormechanismus 15, 16 am oder im Griffstück ausschließlich axial antreibbar ist;
wobei das innere Betätigungsteil 13 an seinem distalen Endabschnitt 133 derart mit dem Gelenk-/Schwenkteil 12 gekoppelt ist, dass eine am proximalen Getriebe-/Aktuatormechanismus 15, 16 vorzugsweise manuell induzierte/ausgelöste/bewirkte axiale Bewegung des inneren Betätigungsteils 13 eine Schwenkung des Gelenk- /Schwenkteils 12 um die Drehachse A bewirkt;
und wobei das Schaftrohr 11 an seinem proximalen Endabschnitt 119 ein Außen- /Spindelgewinde 114 aufweist, das mit einer Spindelmutter 15 in Eingriff ist, welche ein Aktuatorelement des Getriebe-/Aktuatormechanismus 15, 16 ist oder bildet und welche über einen relativ drehbar aber axialfest an der Spindelmutter 15 gehaltenen Mittnehmer 16 als weiteres Aktuatorelement des Getriebe-/Aktuatormechanismus 15, 16 mit dem Betätigungsteil 13 gekoppelt ist.

Bevorzugt ist die Spindelmutter 15 über den Mitnehmer 16 mit dem Betätigungsteil 13 gekoppelt, derart, dass sich bei einer Rotationsbewegung der Spindelmutter 15 um das Schaftrohr 11 der Mitnehmer 16 ausschließlich axial bewegt, wobei der Mitnehmer 16 eine Nase 161 aufweist, welche dazu eingerichtet ist, durch eine längsschlitzförmige Durchgriffsöffnung 116 im Schaftrohr 116 in eine Tasche 136 am/im inneren Betätigungsteil 13 einzugreifen, um bei einer axialen Bewegung des Mitnehmers 15 längs der längsgeschlitzten Durchgriffsöffnung 116 ausschließlich eine axiale Bewegung des Betätigungsteils 13 innerhalb des Schaftrohrs 11 zu bewirken.

Weiter bevorzugt ist die Spindelmutter 15 an ihrer Außenseite mit einer Zahnradstruktur 157 ausgebildet ist, wobei die Spindelmutter 15 vorzugsweise mehrstückig ausgebildet und zusammengesetzt um das Schaftrohr 11 herum angeordnet ist.

Weiter bevorzugt weist das innere Betätigungsteil 13 an seinem distalen Endabschnitt 133 mindestens ein quer zur Schaftrohr-Längsachse L ausgerichtetes sowie in Radialrichtung des Schaftrohrs 12 bzw. des inneren Betätigungsteils 13 gelängtes/aufgeweitetes Langloch 134 auf, durch welches eine mit dem Gelenk-/Schwenkteil 12 gekoppelte r Achse/Stift geführt ist, welcher radial von der Schaftrohr-Längsachse L als auch axial von der Drehachse A des Gelenkteils 12 beabstandet ist und zur Drehachse A parallel ausgerichtet ist.

Weiter bevorzugt ist das Gelenk-/Schwenkteil 12 an seinem dem Schaftrohr 11 zugewandten Endabschnitt längsgeschlitzt, wodurch zwei radial beabstandete, proximale Längsarme 121 gebildet sind und die mit dem Gelenk-/Schwenkteil 12 gekoppelte Achse durch zumindest einen transversal angeordneten zylindrischen Stift 135 gebildet ist, welcher durch eine radial ausgerichtete Durchgangsbohrung 122 in zumindest einem der zwei proximalen Arme 121 des Gelenkteils 12 hindurchgeführt ist.

Vorzugsweise weist das innere Betätigungsteil 13 zumindest abschnittsweise mindestens eine entlang seiner Längsachse L verlaufende Ausnehmung, insbesondere außenseitige Längsnut 131, 132, auf, wobei weiter vorzugsweise in der mindestens einen Ausnehmung, insbesondere außenseitige Längsnut 131, 132, ein Kraftübertragungselement, insbesondere Zugband für eine Betätigung eines Maulteils des Instruments 1, und/oder eine elektrische Versorgungs- und/oder Signalleitung, und/oder eine Klinge angeordnet ist.

Weiter bevorzugt stell die entsprechende Ausnehmung, insbesondere Längsnut 131, 132, eine Führung für das in der mindestens einen Ausnehmung, insbesondere Längsnut, angeordnete Zugband und/oder die in der mindestens einen Ausnehmung, insbesondere Längsnut 131, 132, angeordnete elektrische Versorgungs- und/oder Signalleitung und/oder die in der mindestens einen Ausnehmung, insbesondere Längsnut 131, 132, angeordnete Klinge dar.

Weiter bevorzugt weist das Instrument 1 außerdem an einem distalen Ende des Gelenkteils 12 eine Funktionseinheit, und an dem proximalen Ende 119 des Schaftrohrs 11 eine Handhabungseinheit auf, wobei die Funktionseinheit mittels der Handhabungseinheit betätigbar ist, wobei es sich bei der Funktionseinheit insbesondere um ein Schneid- und/oder Versiegelungswerkzeug und/oder um ein Maulteil handelt.

Das chirurgische/medizinische Instrument 1, hier beispielhaft ein laparoskopisches Instrument, weist im Konkreten einen Instrumentenschaft, vorliegend ein Schaftrohr 11 auf, welches gelenkig / scharnierförmig mit einem Gelenkteil / Instrumentenkopf 12 gekoppelt ist. Das Gelenkteil / der Instrumentenkopf 12 ist hierbei an einem distalen Ende/Endabschnitt (siehe distale Richtung D in Fig. 1) des Schaftrohrs 11 angeordnet, und das Schaftrohr 11 wiederum an einem proximalen Ende/Endabschnitt (siehe proximale Richtung P in Fig. 1) des Gelenkteils 12.

Das distale Ende (Endabschnitt) des Schaftrohrs 11 endet in (bildet) zwei parallelen Armen 111 des Schaftrohrs 11, in denen jeweils eine kreisförmige Öffnung 112 angeordnet ist. **In** anderen Worten ausgedrückt, ist das Schaftrohr 11 an seinem distalen Endabschnitt gegabelt, wodurch zwei parallelbeabstandete Arme oder Laschen 111 entstehen, welche vorzugsweise federelastisch vergleichbar mit einer Federzunge biegbar/radial nach Außen aufweitbar sind. Jeder dieser Arme 111 ist mit einer quer zur Schaftrohrachse L ausgerichteten Durchgangsbohrung versehen, deren Bohrungsachsen A fluchten und welche zur Aufnahme von Scharnierbolzen (Stifte, Zapfen) vorgesehen sind.

Fig. 2 zeigt das proximale Ende (Endabschnitt) des Gelenkteils/Instrumentenkopfs 12 in einer weiteren schematischen dreidimensionalen Ansicht (Perspektivenansicht). Darin ist sichtbar, dass auch das proximale Ende (Endabschnitt) des Gelenkteils 12 in zwei parallelen Armen 121 des Gelenkteils 12 endet, an deren Außenseite jeweils ein runder Außenstift 122 ausgebildet ist. **In** anderen Worten ausgedrückt besteht der Instrumentenkopf aus einer Hülse, deren proximaler Endabschnitt durch eine axiale Einkerbung in zwei parallelbeabstandete Arme, Schenkel oder Laschen 121 geteilt ist, welche vorzugsweise federelastisch vergleichbar mit einer Federzunge biegbar sind. An jeder dieser Laschen 121 ist ein radial nach außen vorragender Stift oder Bolzen 122 ausgeformt, deren zueinander fluchtende Mittenachsen die Längsachse der Hülse rechtwinklig schneiden. Des Weiteren ist an jeder Lasche 121 jeweils eine Durchgangsbohrung 123 vorgesehen, die ebenfalls zueinander fluchtend ausgerichtet und bezüglich des jeweiligen Bolzens 122 außermittig sowie in Richtung proximal versetzt sind, sodass sich zwischen der jeweiligen Durchgangsbohrung 123 und dem zugehörigen Bolzen 122 ein Hebelarm ergibt.

Die Dimensionen der Arme 111 des Schaftrohrs 11 und der Arme 121 des Gelenkteils 12 sind derart gewählt, dass die Arme 111 des Schaftrohrs 11 radial außenseitig über die Arme 12 des Gelenkteils 12 und die beiden Außenstifte 122 geführt werden können, indem sie dabei moderat und elastisch auseinandergebogen werden. Die Abmessungen der kreisförmigen Öffnungen 112 an den Armen 111 des Schaftrohrs 11 sind so gewählt, dass bei entsprechender Überlagerung die runden Außenstifte / Bolzen 122 in die Öffnungen 122 (federelastisch) einschnappen oder einrasten und somit das Gelenkteil 12 durch die Arme 111 des Schaftrohrs 11 gelenkig/scharnierartig gehalten wird. Mit anderen Worten ist der Abstand der beiden Arme 111 des Schaftrohrs so groß wie das Außenmaß des Gelenkstücks 12 an der entsprechenden Stelle, und die Arme 111 sind elastisch verformbar ausgeführt. Auf die oben beschriebene Weise ist das Gelenkteil 12 nahezu spielfrei gesichert und lässt nur noch eine Rotation/Schwenkbewegung des Gelenkteils 112 um eine Drehachse A (oder: Artikulationsachse) zu, welche gemeinsam durch die Außenstifte / Bolzen 122 definiert wird.

Durch die runden Außenstifte 122 und die kreisförmigen Öffnungen 112 ist ein Drehgelenk realisiert, mittels welchem das Gelenkteil 12 gegenüber dem Schaftrohr 11 schwenkbar (oder: artikulierbar) ist, und zwar um eine Drehachse A, welche konzentrisch mit den Außenstiften 122 und den kreisförmigen Öffnungen 112 angeordnet ist.

In Fig. 2 ist außerdem gut zu sehen, dass durch jeden der beiden Arme 121 des Gelenkteils 12 hindurch die kreisförmige Durchgangsbohrung 123 ausgebildet ist, wobei die beiden Durchgangsbohrungen 123 miteinander fluchten. Jede kreisförmige Durchgangsbohrung 123 ist von dem ihr benachbarten Außenstift 122 beabstandet. Der Kreismittelpunkt der jeweiligen Durchgangsbohrung 123 liegt dabei näher an dem proximalen Ende des Gelenkteils 12 als der Kreismittelpunkt des jeweiligen Außenstifts 122. Außerdem ist die jeweilige Durchgangsbohrung 123 bezüglich des jeweiligen Außenstifts 122 auch in einer Richtung versetzt, welche senkrecht auf der Drehachse A sowie senkrecht auf einer Längsachse des Gelenkteils 12 steht. In Fig. 2 ist die Durchgangsbohrung 123 daher gegenüber dem Außenstift 122 beispielsweise, außer nach links, auch noch nach unten versetzt.

In Fig. 1 wiederum ist zu sehen, dass das Instrument 1 außerdem ein separat zum Schaftrohr 11 ausgebildetes Innenteil (Einsatz) 13 (oder, engl. "Inlay") aufweist. Dieses Innenteil 13 ist teilweise innerhalb des Schaftrohrs 11, sowie teilweise innerhalb des Gelenkteils 12 angeordnet, genauer gesagt: zwischen den beiden Armen 121 des Gelenkteils 12, welche wiederum von den beiden Armen 111 des Schaftrohrs 11 umfangen werden. Wie in Fig. 1 auch ersichtlich ist, kann der Raum zwischen den beiden Armen 121 des Gelenkteils 12 im Längsschnitt eine Abrundung, oder anders ausgedrückt, einen kreissegmentförmigen Abschluss, aufweisen, in welchem ein distales Ende des Innenteils 13 aufgenommen ist, sodass das Innenteil 13 die Schwenkung des Gelenkteils 12 bezüglich des Schaftrohrs 11 nicht behindert.

In anderen Worten ausgedrückt ist es für die Funktion des Instrumentenkopfs selbst (nämlich Nickbewegung) als auch für die Funktion des Effektors (nämlich eine Öffnungs- und Schließbewegung insbesondere Zangenbewegung sowie Beaufschlagung mit elektrischem Strom) erforderlich, dass mechanische Kräfte sowie elektrische Energie zuverlässig und präzise an den Instrumentenkopf herangeführt werden. Dabei steht als Raum zur Verlegung entsprechender Leitungen und Kraftübertragungszüge im Wesentlichen nur der vom Schaftrohr gebildete Hohlraum zur Verfügung.

Aus diesem Grund sieht die vorliegende Offenbarung die Verwendung des Innenteils (innere Betätigungsteil) 13 vor. Hierbei handelt es sich im Wesentlichen um einen vorzugsweise vollprofilierten Zug-/Druckstab, der in dem Schaftrohr längsverschiebbar geführt ist (das Innenteil liegt umfangsseitig zumindest abschnittsweise an der Innenwand des Schaftrohrs gleitend an) und der eine Anzahl von längs sich erstreckenden Außennuten aufweist, welche als Führungsnuten/Führungskanäle für elektrische Leitungen und Seilzüge dienen. Das Innenteil (Inlay) in Form eines Zug-/Druckstabs ist somit im Prinzip eine Art Führungshilfe für die Leitungen und Kraftübertragungszüge zur Gewährleistung einer zuverlässigen und präzisen Betätigung/Aktuierung der Funktionen des Instrumentenkopfs bzw. dessen Effektors und weist gleichzeigt auch eine quasi Smartfunktion oder Zusatzfunktion auf, nämlich das Innenteil stellt den Kraftübertragungszug für die Betätigung des Instrumentenkopfs bzw. dessen Nickbewegung selbst dar, welcher wiederum vom Schaftrohr geführt wird und daher präzise eine Betätigungskraft vom Handgriff zum Instrumentenkopf leitet. Zu diesem Zweck erstreckt sich das Innenteil bevorzugt vom Handgriff bis zum Instrumentenkopf und ist dort mit diesem derart wirkverbunden, dass eine Axialverschiebung des Innenteils bezüglich der Rohrschafts in eine Schwenk-/Kipp-/Nickbewegung des Instrumentenkopfs transformiert wird.

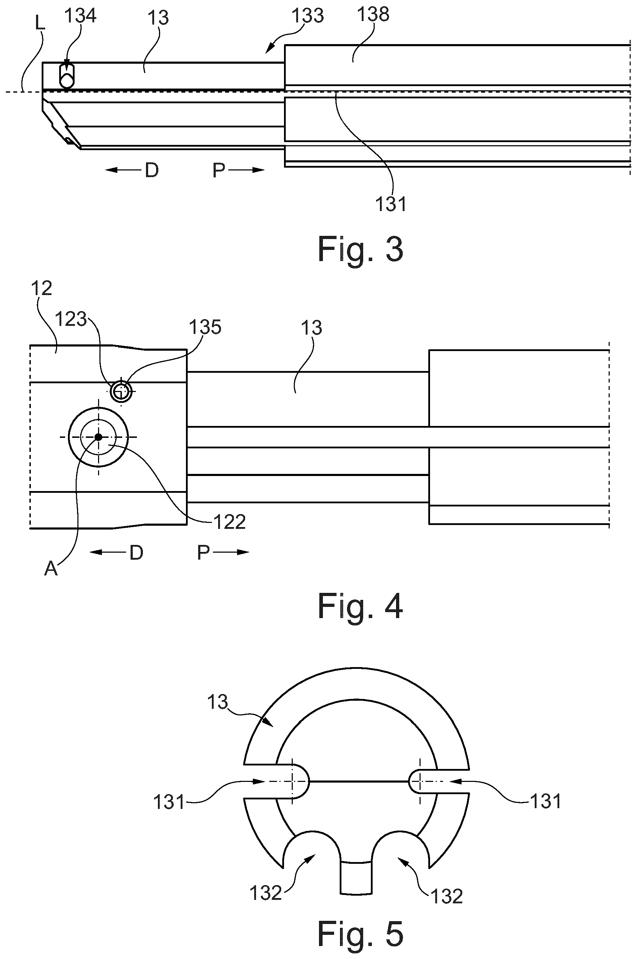

Fig. 3 illustriert schematisch das distale Ende/Endabschnitt 133 des Innenteils 13 im Detail. In Fig. 3 ist demnach ersichtlich, dass das distale Ende/Endabschnitt 133 des Innenteils 13 abgesetzt ausgebildet ist, d.h. insbesondere mit kleinerem Querschnitt als der vorangehende (proximale), mittlere Abschnitt 138 des Innenteils 13. Der mittlere Abschnitt 138 des Innenteils 13, welcher beispielsweise über 80% oder über 90% der Gesamtlänge des Innenteils 13 ausmachen kann, weist vorteilhaft einen Außenumfang auf, welcher nur geringfügig kleiner ist als der Innenumfang des Schaftrohrs 11, sodass sich das Innenteil 13 innerhalb des Schaftrohrs 11 in axialer Richtung frei bewegen kann, in radialer Richtung jedoch durch das Schaftrohr 11 gleitend geführt ist.

In dem distalen Ende/Endabschnitt 133 des Innenteils 13 ist ein quer zur Längsachse L des Innenteils 13 verlaufendes Durchgangsloch ausgebildet, welches bezüglich der Längsachse L radial nach versetzt angeordnet ist und dabei ein Langloch bildet, das sich in einer Richtung quer zur Längsachse (vorzugsweise Tangentialrichtung) erstreckt. Die Breite des Langlochs entspricht in etwa dem Durchmesser der Durchgangsbohrungen 123, welche an den Laschen 121 des Gelenkteils 12 (Instrumentenkopfs) ausgeformt sind. Des Weiteren ist die Stirnseite des Innenteils 13 angeschrägt und erstreckt sich ausgehend von der distalen Endspitze des Innenteils im Bereich des Langlochs schräg nach proximal.

Fig. 4 zeigt schematisch - unter Ausblendung des Schaftrohrs 11 - die gelenkige Verbindung zwischen Gelenkteil 12 und Innenteil 13. Durch die beiden Durchgangsbohrungen 123 und durch das mindestens eine Langloch 134 in dem in das hülsenförmige Gelenkteil 12 eingesetzten Innenteil 13 hindurch ist ein z. B. zylindrischer Stift 135 geführt. Ferner sind die Bolzen 122 in die Bohrungen 112 des Schaftrohres 11 eingerastet, wie dies in der Fig. 1 gezeigt ist. In Konstruktionslage gemäß der Fig. 1 erstecken sich das Schaftrohr 11 und das hülsenförmige Gelenkteil 12 im Wesentlichen koaxial. In dieser Ausrichtung verläuft die schräg nach proximal geneigte distale Stirnseite zwischen dem Scharnierstift 135 und den Bolzen 122, wie dies auch in der Fig. 1 angedeutet ist. Durch die Hebelwirkung infolge des Radialabstands von Bolzen 122 und Scharnierstift 135 kann durch eine rein axiale Bewegung des Innenteils 13 innerhalb des Schaftrohrs 11 das an dem Schaftrohr 11 angelenkte Gelenkteil 12 geschwenkt (oder: artikuliert) werden, indem durch Zug an oder Druck auf das Innenteil 12 über den Stift 135 und das mindestens eine Langloch 134 ein Drehmoment am Gelenkteil 13 erzeugt wird. Wird das Innenteil 13 beispielsweise axial in proximale Richtung P bezüglich dem Schaftrohr 11 bewegt, wird der Stift 135 durch das Langloch 134 (nach rechts in Fig. 4) in Richtung proximal gezogen und somit, aufgrund der versetzten Anordnung (oder: aufgrund der exzentrischen Platzierung) von Durchgangsbohrungen 123 und der Drehachse A zueinander, ein Drehmoment im Uhrzeigersinn auf das Gelenkteil 12 ausgeübt. Auf diese Weise ist das Gelenkteil 12 präzise und akkurat schwenkbar (oder: artikulierbar), da nur ein einziges (ggf. toleranzbehaftetes) Gelenk/Scharnier im Kraftübertragungszug zwischen Gelenkteil und Innenteil vorgesehen ist. Das Langloch 134 dient ferner dazu die Radialbewegung der Durchgangsbohrungen 123 bzw. des Scharnierstifts 135 auszugleichen.

Bevor im Folgenden noch auf die bevorzugten Mechaniken zur axialen Aktuierung des Innenteils 13 eingegangen wird, werden noch die vorteilhaften Ausgestaltungen des Innenteils 13, insbesondere bezüglich dessen Querschnitts, beschrieben.

Wie eingangs bereits ausgeführt wurde, befinden sich an dem distalen Ende des Gelenkteils 12 typischer Weise Funktionseinheiten (Effektoren), zu deren Einführung und Handhabung das Instrument 1 eingesetzt wird. Bei einer solchen Funktionseinheit kann es sich beispielsweise um ein Maulteil, ein Schneid- und/oder Versiegelungswerkzeug, und/oder dergleichen mehr handeln. Die jeweilige Funktionseinheit, oder die Funktionseinheiten, werden proximal, von außerhalb des Körpers des Patienten, an dem Handgriff aktuiert. Dies kann durch elektrische Versorgungs- und/oder Steuersignale erfolgen, die einen oder mehrere Elektromotoren (oder andere elektrische oder elektronische Elemente) der Funktionseinheit mit elektrischem Strom versorgen und/oder steuern. Bei den elektrischen Versorgungs- und/oder Steuersignalen kann es sich um Gleichstrom und/oder um Wechselstrom handeln.

Andererseits kann auch eine direktere, haptisch rückgekoppelte Aktuierung vorgesehen sein. Dafür können sich Kraftübertragungselemente, wie beispielsweise Zugbänder/Zugseile, von einem proximalen Aktuatorelement am Handgriff bis hin zu der distalen Funktionseinheit (Effektor) erstrecken. Ein Benutzer kann ein solches Kraftübertragungselement manuell aktuieren oder einen Motor am Handgriff aktuieren, der wiederum das Kraftübertragungselement betätigt, wobei der Benutzer insbesondere bei rein manueller Betätigung beispielsweise anhand des gespürten Widerstands eine unmittelbare Rückkopplung über das Geschehen an der distalen Funktionseinheit erhält. Insbesondere im letztgenannten Fall ist es erforderlich, dass diese Rückkopplung möglichst präzise und ohne zeitliche Verzögerung erfolgt um ein schwammiges Betätigungsgefühl zu vermeiden.

Fig. 5 zeigt eine schematische Querschnittsdarstellung durch das Innenteil 13, und zwar in dessen mittlerem Abschnitt 138, d.h. zwischen dessen proximalen Ende und dem distalen Ende 133. Die Querschnittsfläche des Innenteils 13 insgesamt ist grob kreisförmig, wobei ausgehend von der umfänglichen Außenseite dieser Kreisform verschiedene Ausnehmungen/Längsnuten gebildet sind. Das Innenteil 13 weist beispielsweise an seiner Außenseite zwei laterale, in einer gemeinsamen Ebene liegende schlitzförmige Längsnuten 131 auf. Durch diese Ebene wird der Querschnitt des Innenteils 13 virtuell in zwei Hemisphären geteilt (in Fig. 5: oben und unten). In einer dieser Hemisphären (in Fig. 5: unten) sind zwei weitere Ausnehmungen in Form von bauchigen Längsnuten 132 ausgebildet. Die schlitzförmigen Längsnuten 131 und die bauchigen Längsnuten 132 sind derart angeordnet, dass eine (in Fig. 5 senkrechte) Spiegelsymmetrieachse bzw. Spiegelsymmetrieebene (entlang der Längsachse L des Innenteils 13) gezogen werden kann.

In den Längsnuten 131, 132 können nun beispielsweise eine Klinge bzw. ein Betätigungsstrang zur Axialverschiebung einer im Instrumentenkopf gelagerten Klinge, ein Kraftübertragungselement (z.B. ein Zugband) vorzugsweise zur Betätigung des Effektors (beispielsweise ein Öffnen und Schließen der Gewebe-Eingriffsbranchen), eine elektrische Versorgungs- und/oder Signalleitung (beispielsweise für die Elektroden an den Branchen) und/oder dergleichen angeordnet werden. Das Innenteil 13 stellt somit für die aufgenommenen Elemente (Leitungen und Kraftübertragungszüge) auch eine Führung bereit, die somit platzsparend, einfach, und mit geringem Aufwand realisiert wird. Beispielsweise kann in den beiden schlitzförmigen Längsnuten 131 jeweils ein Zugband angeordnet sein, sodass die Zugbänder eine symmetrische Kraft auf das Maulteil bzw. dessen Branchen ausüben können. Die bauchigen Längsnuten 132 können beispielsweise für elektrische Versorgungs- und/oder Steuerleitungen vorgesehen sein. Auf diese Weise wird verhindert, dass die Leitungen und Züge ungeordnet im Schaftrohr 11 verlaufen und dadurch auch unnötig hohe Reibungswiderstände entstehen, die eine Betätigungshysterese bewirken können.

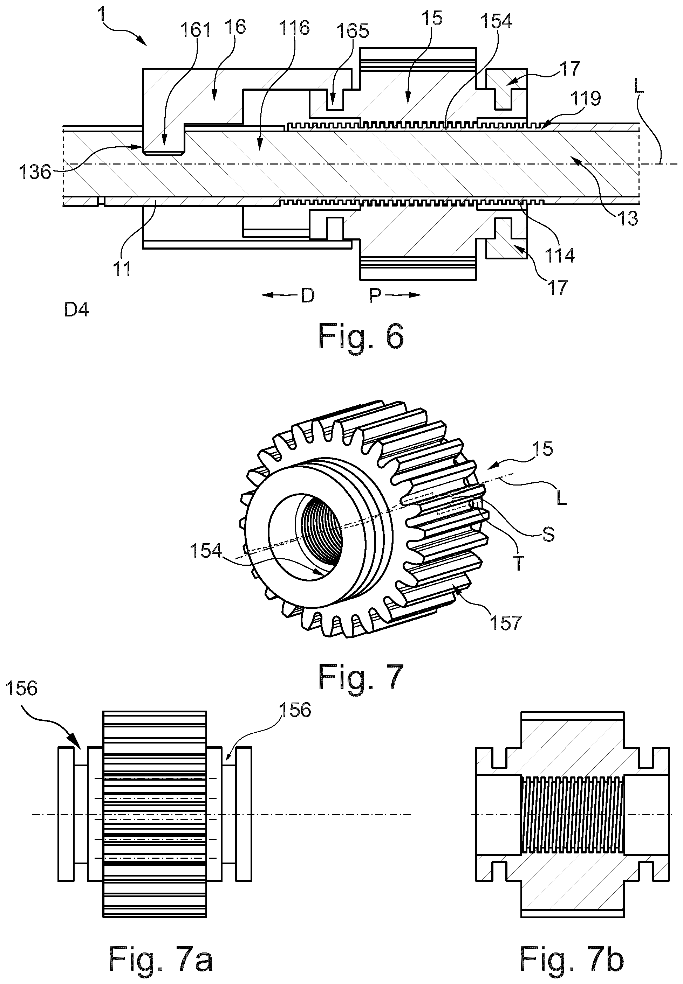

Fig. 6 illustriert in einer schematischen Längsschnittdarstellung die proximale Aktuierung des Innenteils 13 zu dessen axialer Bewegung, und somit auch die Schwenkung des Gelenkteils 12 bezüglich des Schaftrohrs 11, mittels des zuvor beschriebenen proximalen Aktuatormechanismus. Vorzugsweise zeigt die Fig. 6 zumindest einen Teil des Handgriffs des chirurgischen Instruments gemäß der Offenbarung,

Bevorzugt wird die axiale Bewegung des Innenteils 13 platzsparend durch eine rotatorische Bewegung eines hülsenförmigen Aktuatorelements (Spindelmutter) 15 des Instruments 1 bewirkt.

Hierzu weist das Schaftrohr 11 an seinem proximalen Ende/Endabschnitt 119 ein Außengewinde (Gewindespindel) 114 auf, in welches ein Innengewinde 154 des Aktuatorelements 15 eingreift bzw. auf dieses aufgeschraubt ist. Das Aktuatorelement 15 ist somit konzentrisch bezüglich des Schaftrohrs 11 angeordnet und umfasst dieses vollständig an dessen proximalem Endabschnitt. Das Außengewinde 114 ist vorzugsweise in das Schaftrohr 11 hineingeschnitten. Alternativ kann das Außengewinde 114 jedoch auch, beispielsweise in Form einer Hülse, auf das Schaftrohr 11 aufgesteckt sein.

Das Aktuatorelement 15 ist bevorzugt an seiner Außenseite als ein Zahnrad (oder: mit einer Zahnradstruktur 157) ausgebildet, wie besonders gut in Fig. 7 erkennbar ist. Das Aktuatorelement 15 kann alternativ auch ohne Zahnradstruktur 157 ausgebildet werden und dann beispielsweise zur händischen Rotation ausgebildet sein.

Fig. 7 zeigt eine schematische dreidimensionale Ansicht des hier beispielsweise in seiner Außenkontur als Zahnrad ausgebildeten Aktuatorelements 15. Die Zahnradfurchen der Zahnradstruktur 157 sind entlang der Längsachse des Schaftrohrs 11 ausgebildet, welche identisch mit der Längsachse L des Innenteils 13 ist. Somit kann beispielsweise das Betreiben eines Antriebsmotors, der mit dem Zahnrad in Wirkeingriff ist, eine rotatorische Bewegung dieser Zahnradstruktur 157 bewirken, welche sich aufgrund des Außengewindes 154 und des Innengewindes 114 dann axial entlang des Schaftrohrs 11 bewegt.

Wie in Fig. 7 außerdem zu sehen ist, weist das Aktuatorelement 15, in Längsrichtung jeweils vor und nach der Zahnradstruktur 157, eine umlaufende Ringnut 156 auf.

Das Aktuatorelement 15 ist bevorzugt mehrteilig ausgebildet, zum Beispiel zweiteilig. In Fig. 7 ist beispielhaft eine Trennlinie T zwischen den zwei Bestandteilen des Aktuatorelements 15 ersichtlich, die vorteilhaft uneben, d.h., nicht innerhalb einer Ebene, verläuft, um das Aktuatorelement 15 gegen Verschiebungen und dergleichen robuster zu machen. Bevorzugt umfasst die Trennlinie T hierzu eine Stufe S. Die Gesamtstruktur des Aktuatorelements 15 kann bevorzugt durch in den umlaufenden Ringnuten 156 angeordnete kreisförmige Strukturen zusammengehalten werden, was eine einfache Montage ermöglicht.

Alternativ kann das Aktuatorelement 15 auch einstückig ausgebildet werden. In diesem Fall kann es an einer Seite geschlitzt ausgebildet sein. Zur Montage kann es an dem Schlitz aufgeweitet und auf das Schaftrohr 11 aufgesteckt werden. Ist das Außengewinde 114 nur in Form einer Hülse auf das Schaftrohr 11 aufgesteckt, kann auch das Aktuatorelement 15 einteilig und ungeschlitzt ausgebildet sein.

In Fig. 6 ist hierzu dargestellt, dass in die proximal angeordnete umlaufende Ringnut 156 ein Sicherungsring 17 eingreift oder eingesetzt ist, der die zwei Bestandteile des Aktuatorelements 15 zusammenhält. Die distal angeordnete umlaufende Ringnut 156 ist mit einem Mitnehmer 16 des Instruments 1 gekoppelt.

Auch der Mitnehmer 16 ist konzentrisch zu dem Schaftrohr 11 angeordnet und umfasst dieses. Er weist einen radial nach innen sich erstreckenden Vorsprung, nämlich einen Ringvorsprung 165 auf, welcher dazu ausgebildet und angeordnet ist, in die distale umlaufende Ringnut 156 des Aktuatorelements 15 einzugreifen. Demnach ist der Mitnehmer 16 also nicht nur auf das Schaftrohr 11, sondern auch auf das Aktuatorelement 15 aufgesteckt. Auf diese Weise wird nicht nur die (ggfs.) mehrteilige Gesamtstruktur des Aktuatorelements 15 zusätzlich zusammengehalten, sondern die axiale Bewegung des Aktuatorelements 15 auch auf den Mitnehmer 16 übertragen. Das Aktuatorelement 15 und der Mitnehmer 16 (und ggfs. weitere Elemente) bilden somit einen proximalen Aktuatormechanismus.

Der Mitnehmer 16 wiederum weist eine radial nach innen vorstehende Nase 161 auf, welche durch eine radiale (oder: laterale), in Längsrichtung sich erstreckende Aussparung (Schlitz) 116 in dem Schaftrohr 11 in eine radiale (oder: laterale) Tasche 136 (oder: Ausnehmung) in dem Innenteil 13, welches in das Schaftrohr 11 eingelegt ist, eingreift, insbesondere formschlüssig. Die Aussparung 116 in dem Schaftrohr 11 ist bevorzugt (nur) geringfügig breiter als die Nase 161 des Mitnehmers 16, sodass eine Rotation des Mitnehmers 16 gegenüber dem Schaftrohr 11 verhindert wird. Der Mitnehmer 16 kann somit ausschließlich eine axiale Bewegung bezüglich des Schaftrohrs 11 ausführen. Die Kopplung zwischen dem Mitnehmer 16 und dem Aktuatorelement 15 über die Ringnut 156 und den Ringvorsprung 165 ist derart realisiert, dass das Aktuatorelement 16 sich gegenüber dem Mitnehmer 16 drehen kann.

Die Aussparung 116 in dem Schaftrohr 11 sowie der Mitnehmer 16 sind distalseitig von dem Außengewinde 114 des Schaftrohrs 11 angeordnet. Die axiale Bewegung des Mitnehmers 16 wird somit durch die Nase 161 und die Tasche 136 auf das Innenteil 13 übertragen, welches entsprechend ebenfalls eine axiale Bewegung durchführt. Somit kann mittelbar durch Einbringen einer rotatorischen Bewegung auf das Aktuatorelement 15 das Innenteil axial verschoben und damit das Gelenkteil 12 bezüglich des Schaftrohrs verschwenkt (oder: artikuliert) werden. Aufgrund der Selbsthemmung der Gewindeverbindung 114, 154 ist die Rückstellung des Winkels des Gelenkteils 12 durch Krafteinwirkung auf das Gelenkteil 12 ausgeschlossen. Das Aktuatorelement 15 muss somit nicht rotatorisch gesichert werden, um die Winkelposition des Gelenkteils 12 zu halten.

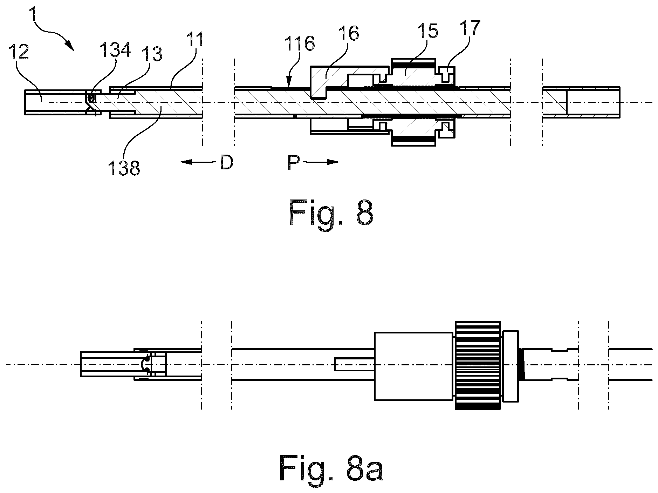

Fig. 8 schließlich zeigt eine schematische Gesamtansicht des Instruments 1 inklusive der distalen Artikulationsmechanik gemäß Fig. 1-4 sowie der proximalen Aktuatormechanik gemäß Fig.6 und Fig. 7. Das dazwischenliegende Mittelstück des Instruments 1, insbesondere des Schaftrohrs 11 und des Innenteils 13, welches in Längsrichtung bevorzugter Weise ca. 4-6 Mal länger ist als die dargestellten Elemente zusammen, wurde aus Darstellungsgründen entfernt. In distaler Richtung D würde sich die Funktionseinheit (nicht dargestellt) anschließen, etwa ein Maulteil oder dergleichen.

Zusammengefasst stellt die Offenbarung ein Instrument 1 für minimalinvasive Chirurgie, vorzugsweise für die Laparoskopie bereit, zumindest aufweisend:
ein Schaftrohr 11;
ein Gelenkteil 12, welches an einem distalen Ende des Schaftrohrs 11 derart gelenkig mit dem Schaftrohr 11 verbunden ist, dass das Gelenkteil 12 bezüglich des Schaftrohrs 11 um eine Drehachse A schwenkbar ist;
einen proximalen Aktuatormechanismus (15, 16); und
ein Innenteil 13, welches teilweise in das Schaftrohr 11 eingesetzt ist und über den proximalen Aktuatormechanismus 15, 16 innerhalb des Schaftrohrs 11 axial bewegbar ist;
wobei das Innenteil 13 an seinem distalen Ende 133 derart mit dem Gelenkteil 12 gekoppelt ist, dass eine axiale Bewegung des Innenteils 13 eine Schwenkung des Gelenkteils 12 um die Drehachse A bewirkt.

### Bezugszeichenliste

### Laparoskopisches Instrument

- 11: Schaftrohr
- 12: Gelenkteil
- 13: Innenteil
- 15: Aktuatorelement
- 16: Mitnehmer
- 17: Sicherungsring
- 111: Distale Arme des Schaftrohrs
- 112: Öffnung
- 114: Außengewinde des Schaftrohrs
- 116: Aussparung in dem Schaftrohr
- 119: proximales Ende des Schaftrohrs
- 121: Proximale Arme des Gelenkteils
- 122: Außenstifte des Gelenkteils
- 123: Durchgangsbohrung
- 131: schlitzförmige Längsnut in dem Innenteil
- 132: bauchige Längsnut in dem Innenteil
- 133: distales Ende des Innenteils
- 134: Langloch
- 135: Stift
- 136: Tasche in dem Innenteil
- 138: Mittlerer Abschnitt des Innenteils
- 154: Innengewinde des Aktuatorelements
- 156: umlaufende Ringnuten des Aktuatorelements
- 157: Zahnradstruktur
- 161: Nase des Mitnehmers
- 165: Ringvorsprung des Mitnehmers

- A: Drehachse
- D: Distale Richtung
- L: Längsachse
- P: Proximale Richtung
- S: Stufe
- T: Trennlinie

## Patentansprüche

1. Instrument (1) für minimalinvasive Chirurgie, vorzugsweise für die Laparoskopie, aufweisend:
ein Schaftrohr (11);
ein Gelenkteil (12), welches an einem distalen Ende des Schaftrohrs (11) derart gelenkig mit dem Schaftrohr (11) verbunden ist, dass das Gelenkteil (12) bezüglich des Schaftrohrs (11) um eine Drehachse (A) schwenkbar ist;
einen proximalen Aktuatormechanismus (15, 16); und
ein Innenteil (13), welches zumindest teilweise in das Schaftrohr (11) derart eingesetzt ist, dass die Innenwand des Schaftrohrs (11) eine Axialgleitführung für das Innenteil (13) bildet und über den proximalen Aktuatormechanismus (15, 16) ausschließlich axial antreibbar ist;
wobei das Innenteil (13) an seinem distalen Ende (133) derart mit dem Gelenkteil (12) gekoppelt ist, dass eine angetriebene axiale Bewegung des Innenteils (13) eine Schwenkung des Gelenkteils (12) um die Drehachse (A) bewirkt;
**dadurch gekennzeichnet, dass**
das Schaftrohr (11) an einem proximalen Ende (119) ein Außengewinde (114) aufweist, in welches ein Innengewinde (154) eines Aktuatorelements (15) des Aktuatormechanismus (15, 16) eingreift.

2. Instrument (1) nach Anspruch 1,
wobei das Aktuatorelement (15) mit einem Mitnehmer (16) des Instruments (1) derart gekoppelt ist, dass sich bei einer Rotationsbewegung des Aktuatorelements (15) um das Schaftrohr (11) der Mitnehmer (16) axial bewegt, wobei der Mitnehmer (16) eine Nase (161) aufweist, welche dazu eingerichtet ist, durch eine Öffnung (116) in dem Schaftrohr (116) in eine Tasche (136) in dem Innenteil (13) einzugreifen und bei einer axialen Bewegung des Mitnehmers (15) eine axiale Bewegung des Innenteil (13) zu bewirken.

3. Instrument (1) nach Anspruch 1,
wobei das Aktuatorelement (15) an seiner Außenseite mit einer Zahnradstruktur (157) ausgebildet ist.

4. Instrument (1) nach einem der Ansprüche 1 bis 3,
wobei das Aktuatorelement (15) mehrstückig ausgebildet und zusammengesetzt um das Schaftrohr (11) herum angeordnet ist.

5. Instrument (1) nach einem der Ansprüche 1 bis 4,
wobei das Innenteil (13) an seinem distalen Ende (133) mindestens ein Langloch (134) aufweist, durch welches eine mit dem Gelenkteil (12) gekoppelte Achse geführt ist, welche von der Drehachse (A) des Gelenkteils (12) beabstandet und zu dieser parallel angeordnet ist.

6. Instrument (1) nach Anspruch 5,
wobei die mit dem Gelenkteil (12) gekoppelte Achse durch einen transversal angeordneten zylindrischen Stift (135) gebildet ist, welcher durch eine jeweilige kreisförmige Durchgangsbohrung (122) durch einen jeweiligen Arm (121) von zwei proximalen Armen (121) des Gelenkteils (12) hindurchgeführt ist.

7. Instrument (1) nach einem der Ansprüche 1 bis 6,
wobei das Innenteil (13) zumindest abschnittsweise mindestens eine entlang seiner Längsachse (L) verlaufende Ausnehmung, insbesondere außenseitige Längsnut (131, 132), aufweist.

8. Instrument (1) nach einem der Ansprüche 1 bis 7,
wobei in der mindestens einen Ausnehmung, insbesondere außenseitige Längsnut (131, 132), ein Kraftübertragungselement, insbesondere Zugband für eine Betätigung eines Maulteils des Instruments (1), und/oder eine elektrische Versorgungs- und/oder Signalleitung, und/oder eine Klinge angeordnet ist.

9. Instrument (1) nach Anspruch 8,
wobei die entsprechende Ausnehmung, insbesondere Längsnut (131, 132), eine Führung für das in der mindestens einen Ausnehmung, insbesondere Längsnut, angeordnete Zugband und/oder die in der mindestens einen Ausnehmung, insbesondere Längsnut (131, 132), angeordnete elektrische Versorgungs- und/oder Signalleitung und/oder die in der mindestens einen Ausnehmung, insbesondere Längsnut (131, 132), angeordnete Klinge darstellt.

10. Instrument (1) nach einem der Ansprüche 1 bis 9,
wobei das Instrument (1) außerdem an einem distalen Ende des Gelenkteils (12) eine Funktionseinheit aufweist und an dem proximalen Ende (119) des Schaftrohrs (11) eine Handhabungseinheit aufweist, wobei die Funktionseinheit mittels der Handhabungseinheit betätigbar ist, wobei
wobei es sich bei der Funktionseinheit insbesondere um ein Schneid- und/oder Versiegelungswerkzeug und/oder um ein Maulteil handelt.

## Claims

1. An instrument (1) for minimally invasive surgery, preferably for laparoscopy, comprising:
a shaft tube (11);
a joint part (12) which is connected in an articulated manner to the shaft tube (11) at a distal end of the shaft tube (11) in such a manner that the joint part (12) is pivotable about an axis of rotation (A) with respect to the shaft tube (11);
a proximal actuator mechanism (15, 16); and
an inner part (13) which is inserted at least partially into the shaft tube (11) in such a manner that the inner wall of the shaft tube (11) forms an axial sliding guide for the inner part (13) and can be driven exclusively axially via the proximal actuator mechanism (15, 16);
wherein the inner part (13) is coupled at its distal end (133) to the joint part (12) in such a manner that a driven axial movement of the inner part (13) causes pivoting of the joint part (12) about the axis of rotation (A);
**characterized in that**
the shaft tube (11) comprises, at a proximal end (119), an external thread (114) into which an internal thread (154) of an actuator element (15) of the actuator mechanism (15, 16) engages.

2. The instrument (1) according to claim 1,
wherein the actuator element (15) is coupled to a driver (16) of the instrument (1) in such a manner that the driver (16) moves axially during a rotational movement of the actuator element (15) about the shaft tube (11), wherein the driver (16) comprises a nose (161) which is configured to engage into a pocket (136) in the inner part (13) through an opening (116) in the shaft tube (116) and to cause an axial movement of the inner part (13) during an axial movement of the driver (15).

3. The instrument (1) according to claim 1,
wherein the actuator element (15) is formed on its outer side with a gearwheel structure (157).

4. The instrument (1) according to one of claims 1 to 3,
wherein the actuator element (15) is formed in multiple pieces and is assembled around the shaft tube (11).

5. The instrument (1) according to one of claims 1 to 4,
wherein the inner part (13) comprises, at its distal end (133), at least one elongated hole (134) through which an axis coupled to the joint part (12) is guided, wherein the axis is spaced apart from the axis of rotation (A) of the joint part (12) and is arranged parallel thereto.

6. The instrument (1) according to claim 5,
wherein the axis coupled to the joint part (12) is formed by a transversely arranged cylindrical pin (135) which is guided through a respective circular through-hole (122) through a respective arm (121) of two proximal arms (121) of the joint part (12).

7. The instrument (1) according to one of claims 1 to 6,
wherein the inner part (13) comprises, at least in sections, at least one recess, in particular an outer longitudinal groove (131, 132), running along its longitudinal axis (L).

8. The instrument (1) according to one of claims 1 to 7,
wherein a force transmission element, in particular a pull band for an actuation of a jaw part of the instrument (1), and/or an electrical supply and/or signal line, and/or a blade is arranged in the at least one recess, in particular the outer longitudinal groove (131, 132).

9. The instrument (1) according to claim 8,
wherein the corresponding recess, in particular longitudinal groove (131, 132), constitutes a guide for the pull band arranged in the at least one recess, in particular longitudinal groove, and/or the electrical supply and/or signal line arranged in the at least one recess, in particular longitudinal groove (131, 132), and/or the blade arranged in the at least one recess, in particular longitudinal groove (131, 132).

10. The instrument (1) according to one of claims 1 to 9,
wherein the instrument (1) furthermore comprises a functional unit at a distal end of the joint part (12) and comprises a handling unit at the proximal end (119) of the shaft tube (11), wherein the functional unit can be actuated via the handling unit, wherein
the functional unit is in particular a cutting and/or sealing tool and/or a jaw part.

## Revendications

1. Instrument (1) pour la chirurgie mini-invasive, de préférence pour la laparoscopie, présentant :
un tube de tige (11) ;
une partie articulée (12), laquelle est connectée de manière articulée au tube de tige (11) à une extrémité distale du tube de tige (11) de sorte que la partie articulée (12) peut pivoter par rapport au tube de tige (11) autour d'un axe de rotation (A) ;
un mécanisme d'actionneur proximal (15, 16) ; et
une partie intérieure (13), laquelle est insérée au moins partiellement dans le tube de tige (11) de sorte que la paroi intérieure du tube de tige (11) forme un guide de glissement axial pour la partie intérieure (13) et peut être entraînée exclusivement axialement par le mécanisme d'actionneur proximal (15, 16) ;
dans lequel la partie intérieure (13) est couplée à son extrémité distale (133) à la partie articulée (12) de sorte qu'un mouvement axial entraîné de la partie intérieure (13) provoque un pivotement de la partie articulée (12) autour de l'axe de rotation (A) ;
**caractérisé en ce que**
le tube de tige (11) présente à une extrémité proximale (119) un filetage extérieur (114) dans lequel un filetage intérieur (154) d'un élément d'actionneur (15) du mécanisme d'actionneur (15, 16) s'engage.

2. Instrument (1) selon la revendication 1,
dans lequel l'élément d'actionneur (15) est couplé à un entraîneur (16) de l'instrument (1) de sorte que, lors d'un mouvement de rotation de l'élément d'actionneur (15) autour du tube de tige (11), l'entraîneur (16) se déplace axialement, dans lequel l'entraîneur (16) présente un ergot (161), lequel est configuré pour s'engager dans une poche (136) dans la partie intérieure (13) à travers une ouverture (116) dans le tube de tige (116) et pour provoquer un mouvement axial de la partie intérieure (13) lors d'un mouvement axial de l'entraîneur (15).

3. Instrument (1) selon la revendication 1,
dans lequel l'élément d'actionneur (15) est conçu sur son côté extérieur avec une structure à pignon (157).

4. Instrument (1) selon l'une quelconque des revendications 1 à 3,
dans lequel l'élément d'actionneur (15) est conçu en plusieurs parties et est disposé de manière assemblée autour du tube de tige (11).

5. Instrument (1) selon l'une quelconque des revendications 1 à 4,
dans lequel la partie intérieure (13) présente à son extrémité distale (133) au moins un trou oblong (134) à travers lequel un axe couplé à la partie articulée (12) passe, lequel est espacé de l'axe de rotation (A) de la partie articulée (12) et disposé parallèlement à celui-ci.

6. Instrument (1) selon la revendication 5,
dans lequel l'axe couplé à la partie articulée (12) est formé par une tige cylindrique (135) disposée transversalement, laquelle est guidée à travers un alésage traversant circulaire respectif (122) par un bras respectif (121) de deux bras proximaux (121) de la partie articulée (12).

7. Instrument (1) selon l'une quelconque des revendications 1 à 6,
dans lequel la partie intérieure (13) présente, au moins par sections, au moins un évidement s'étendant le long de son axe longitudinal (L), en particulier une rainure longitudinale extérieure (131, 132).

8. Instrument (1) selon l'une quelconque des revendications 1 à 7,
dans lequel un élément de transmission de force, en particulier une bande de traction pour l'actionneur d'une partie de mâchoire de l'instrument (1), et/ou un câble d'alimentation électrique et/ou de signalisation, et/ou une lame est disposée dans l'au moins un évidement, en particulier la rainure longitudinale extérieure (131, 132).

9. Instrument (1) selon la revendication 8,
dans lequel l'évidement correspondant, en particulier la rainure longitudinale (131, 132), représente un guide pour la bande de traction disposée dans au moins un évidement, en particulier une rainure longitudinale, et/ou le câble d'alimentation électrique et/ou de signalisation disposé dans au moins un évidement, en particulier une rainure longitudinale (131, 132), et/ou la lame disposée dans au moins un évidement, en particulier une rainure longitudinale (131, 132).

10. Instrument (1) selon l'une quelconque des revendications 1 à 9,
dans lequel l'instrument (1) présente en outre une unité fonctionnelle à une extrémité distale de la partie articulée (12) et une unité de manipulation à l'extrémité proximale (119) du tube de tige (11), dans lequel l'unité fonctionnelle peut être actionnée au moyen de l'unité de manipulation,
dans lequel l'unité fonctionnelle est en particulier un outil de coupe et/ou de scellement et/ou une partie de mâchoire.
